(19)

Europäisches
Patentamt
European
Patent Office
Office européen
des brevets

(11) **EP 3 736 288 A1**

(12) **EUROPEAN PATENT APPLICATION**
published in accordance with Art. 153(4) EPC

(43) Date of publication:
**11.11.2020 Bulletin 2020/46**

(21) Application number: **19735980.5**

(22) Date of filing: **04.01.2019**

(51) Int Cl.:
**C07K 16/22** (2006.01)          **C07K 16/24** (2006.01)
**A61K 39/395** (2006.01)          **A61P 27/02** (2006.01)

(86) International application number:
**PCT/CN2019/070479**

(87) International publication number:
**WO 2019/134686 (11.07.2019 Gazette 2019/28)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **05.01.2018 CN 201810011151**

(71) Applicant: **Bio-Thera Solutions, Ltd.
Guangzhou, Guangdong 510530 (CN)**

(72) Inventors:
• **WU, Xiaoyun
Guangzhou, Guangdong 510530 (CN)**
• **XU, Chenchao
Guangzhou, Guangdong 510530 (CN)**
• **WANG, Zhigang
Guangzhou, Guangdong 510530 (CN)**
• **LI, Shengfeng
Guangzhou, Guangdong 510530 (CN)**

(74) Representative: **Rimini, Rebecca et al
Jacobacci & Partners S.p.A.
Corso Emilia, 8
10152 Torino (IT)**

(54) **LONG-ACTING AND LOW-TOXIC RECOMBINANT ANTI-VEGF HUMANIZED MONOCLONAL ANTIBODY AND PRODUCTION METHOD THEREFOR**

(57)     The present invention relates to a full length humanized monoclonal antibody that can specifically bind to VEGF. The antibody can inhibit binding of VEGF to VEGFR-1 and VEGFR-2, thereby inhibiting signaling of VEGF. The antibody has a strong affinity for VEGF, has a long half-life, and is highly safe after vitreous injection. The antibody of the invention may be effective in treating diseases associated with VEGF overexpression, particularly diseases associated with abnormal angiogenesis caused by VEGF overexpression.

FIG. 5

EP 3 736 288 A1

SEQUENCE LISTING

<110>  BIO-THERA SOLUTIONS, LTD.

<120>  LONG-ACTING AND LOW-TOXIC RECOMBINANT ANTI-VEGF HUMANIZED MONOCLONAL ANTIBODY AND PRODUCTION METHOD THEREFOR

<130>  PT20182361

<150>  201810011151.5
<151>  2018-01-05

<160>  2

<170>  PatentIn version 3.3

<210>  1
<211>  453
<212>  PRT
<213>  Artificial Sequence

<220>
<223> heavy chain amino acid sequence of full-length humanized anti-VEGF antibody

<400>  1
```
Glu Val Gln Leu Val Glu Ser Gly Gly Gly Leu Val Gln Pro Gly Gly
1               5                   10                  15
Ser Leu Arg Leu Ser Cys Ala Ala Ser Gly Tyr Asp Phe Thr His Tyr
            20                  25                  30
Gly Met Asn Trp Val Arg Gln Ala Pro Gly Lys Gly Leu Glu Trp Val
            35                  40                  45
Gly Trp Ile Asn Thr Tyr Thr Gly Glu Pro Thr Tyr Ala Ala Asp Phe
        50                  55                  60
Lys Arg Arg Phe Thr Phe Ser Leu Asp Thr Ser Lys Ser Thr Ala Tyr
65                  70                  75                  80
Leu Gln Met Asn Ser Leu Arg Ala Glu Asp Thr Ala Val Tyr Tyr Cys
                85                  90                  95
Ala Lys Tyr Pro Tyr Tyr Tyr Gly Thr Ser His Trp Tyr Phe Asp Val
            100                 105                 110
Trp Gly Gln Gly Thr Leu Val Thr Val Ser Ser Ala Ser Thr Lys Gly
        115                 120                 125
Pro Ser Val Phe Pro Leu Ala Pro Ser Ser Lys Ser Thr Ser Gly Gly
        130                 135                 140
Thr Ala Ala Leu Gly Cys Leu Val Lys Asp Tyr Phe Pro Glu Pro Val
145                 150                 155                 160
Thr Val Ser Trp Asn Ser Gly Ala Leu Thr Ser Gly Val His Thr Phe
                165                 170                 175
Pro Ala Val Leu Gln Ser Ser Gly Leu Tyr Ser Leu Ser Ser Val Val
                180                 185                 190
Thr Val Pro Ser Ser Ser Leu Gly Thr Gln Thr Tyr Ile Cys Asn Val
                195                 200                 205
Asn His Lys Pro Ser Asn Thr Lys Val Asp Lys Lys Val Glu Pro Lys
        210                 215                 220
Ser Cys Asp Lys Thr His Thr Cys Pro Pro Cys Pro Ala Pro Glu Leu
225                 230                 235                 240
Leu Gly Gly Pro Ser Val Phe Leu Phe Pro Pro Lys Pro Lys Asp Thr
                245                 250                 255
Leu Met Ile Ser Arg Thr Pro Glu Val Thr Cys Val Val Val Asp Val
                260                 265                 270
Ser His Glu Asp Pro Glu Val Lys Phe Asn Trp Tyr Val Asp Gly Val
                275                 280                 285
Glu Val His Asn Ala Lys Thr Lys Pro Arg Glu Glu Gln Tyr Asn Ser
```

```
      290                    295                   300
Thr Tyr Arg Val Val Ser Val Leu Thr Val Leu His Gln Asp Trp Leu
305                    310                   315                   320
Asn Gly Lys Glu Tyr Lys Cys Lys Val Ser Asn Lys Ala Leu Pro Ala
                  325                   330                   335
Pro Ile Glu Lys Thr Ile Ser Lys Ala Lys Gly Gln Pro Arg Glu Pro
              340                   345                   350
Gln Val Tyr Thr Leu Pro Pro Ser Arg Glu Glu Met Thr Lys Asn Gln
          355                   360                   365
Val Ser Leu Thr Cys Leu Val Lys Gly Phe Tyr Pro Ser Asp Ile Ala
      370                   375                   380
Val Glu Trp Glu Ser Asn Gly Gln Pro Glu Asn Asn Tyr Lys Thr Thr
385                   390                   395                   400
Pro Pro Val Leu Asp Ser Asp Gly Ser Phe Phe Leu Tyr Ser Lys Leu
                  405                   410                   415
Thr Val Asp Lys Ser Arg Trp Gln Gln Gly Asn Val Phe Ser Cys Ser
              420                   425                   430
Val Met His Glu Ala Leu His Asn His Tyr Thr Gln Lys Ser Leu Ser
          435                   440                   445
Leu Ser Pro Gly Lys
          450
```

```
<210>  2
<211>  214
<212>  PRT
<213>  Artificial Sequence

<220>
<223>  light chain amino acid sequence of full-length humanized anti-VEGF
antibody

<400>  2
Asp Ile Gln Leu Thr Gln Ser Pro Ser Ser Leu Ser Ala Ser Val Gly
1               5                   10                  15
Asp Arg Val Thr Ile Thr Cys Ser Ala Ser Gln Asp Ile Ser Asn Tyr
          20                  25                  30
Leu Asn Trp Tyr Gln Gln Lys Pro Gly Lys Ala Pro Lys Val Leu Ile
      35                  40                  45
Tyr Phe Thr Ser Ser Leu His Ser Gly Val Pro Ser Arg Phe Ser Gly
  50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Tyr Ser Thr Val Pro Trp
              85                  90                  95
Thr Phe Gly Gln Gly Thr Lys Val Glu Ile Lys Arg Thr Val Ala Ala
          100                 105                 110
Pro Ser Val Phe Ile Phe Pro Pro Ser Asp Glu Gln Leu Lys Ser Gly
          115                 120                 125
Thr Ala Ser Val Val Cys Leu Leu Asn Asn Phe Tyr Pro Arg Glu Ala
          130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
              165                 170                 175
Ser Thr Leu Thr Leu Ser Lys Ala Asp Tyr Glu Lys His Lys Val Tyr
          180                 185                 190
Ala Cys Glu Val Thr His Gln Gly Leu Ser Ser Pro Val Thr Lys Ser
          195                 200                 205
Phe Asn Arg Gly Glu Cys
          210
```

## Description

### TECHNICAL FIELD

The present invention belongs to the field of bio-pharmaceuticals. More specifically, the present invention relates to an antibody for reducing human vascular endothelial growth factor (VEGF/VEGF-A), and further relates to a preparation method and use of the antibody.

### BACKGROUND

Angiogenesis is that vascular endothelial cells proliferate from the preexisting vascular network and recombine into new blood vessels. Angiogenesis is essential for normal proliferation processes of the human body, including wound healing and development and differentiation of organs. Moreover, angiogenesis also relates to the development of a variety of pathological diseases, such as age-related macular degeneration, tumors, rheumatoid arthritis and psoriasis. In view of important pathophysiological significance, some people believe that the process of angiogenesis is regulated by the balance between pro-angiogenic molecules and anti-angiogenic molecules and abnormal regulation occurs in diseases. Angiogenesis is a cascade of processes, including the following processes: degradation of extracellular matrices at local positions subsequent to protease release; proliferation of capillary endothelial cells; and migration of capillaries to angiogenic stimulants.

The process of neovascularization is multifactorial and highly complex, but VEGF is considered as the most critical factor in physiological and pathological angiogeneses. VEGF is essential for vasculogenesis and angiogenesis of an embryo. In addition to being an angiogenic factor in angiogenesis and vasculogenesis, VEGF is also a pleiotropic growth factor, showing a variety of biological effects in endothelial cell survival, vascular permeability, vasodilatation, monocyte chemotaxis and calcium influx. For example, it has been reported that VEGF can promote the division of retinal pigment endothelial cells and lemmocytes. Angiogenesis is the formation of new blood vessels by proliferation and recombination of vascular endothelial cells. It has been evidenced that the development of vascular supply is essential for normal and pathological proliferation. A lot of data show that VEGF plays a key role in the development of diseases related to pathological angiogenesis. VEGF mRNA is overexpressed in most of human tumors. The concentration of VEGF in aqueous humor is highly associated with active hyperplasia of vessels found in patients with diabetes or other ischemic retinal diseases, particularly in choroid neovascular membranes of patients with age-related macular degeneration (AMD).

VEGF is a glycosylated secretory polypeptide growth factor and a homodimeric glycoprotein with a molecular weight between 46 kDa and 48 kDa. It directly acts on vascular endothelial cells, and can induce proliferation of vascular endothelial cells and angiogenesis. There are six types of VEGF family members, including VEGF-A, VEGF-B, VEGF-C, VEGF-D, VEGF-E and placental growth factor, wherein VEGF-A is most important in eyes. VEGF-A gene is located on p12-p21 of chromosome 6, and consists of eight exons and seven introns. Due to different mRNA splicing modes, seven protein forms, *i.e.* VEGF-A$_{121}$, VEGF-A$_{145}$, VEGF-A$_{148}$, VEGF-A$_{165}$, VEGF-A$_{183}$, VEGF-A$_{189}$ and VEGF-A$_{206}$, are produced. Five of the protein forms can increase vascular permeability, promote the proliferation of vascular endothelial cells and induce neovascularization. There are three types of VEGF receptors (VEGFR): VEGFR1 (Flt-1), VEGFR2 (KDR) and VEGFR3 (Flt-4), and VEGF exerts its biological effects by binding to these receptors. High level of VEGF expression was found in both an experimental model and the pathological angiogenesis of wet AMD. Moreover, according to a considerable number of literature, neovascularization and vascular leakage can be inhibited by neutralizing VEGF. Finding out that VEGF plays an important role in angiogenesis in pathological conditions, people have taken a lot of measures to block the activity of VEGF. These measures include utilizing inhibitory anti-VEGF receptor antibodies, soluble receptor constructs, antisense strategies and low-molecular weight VEGF receptor tyrosine kinase inhibitors to interfere with VEGF pathways, *etc.* Related literature has reported that anti-VEGF neutralizing antibodies have shown the capability of inhibiting the growth of a variety of human tumor cell lines in nude mice and intraocular angiogenesis in ischemic retinal disease models. Therefore, anti-VEGF monoclonal antibodies or VEGF action inhibitors are effective candidates for treating solid tumors and a variety of intraocular neovascular diseases.

Macular degeneration is a medical condition mainly found in old people, which is known as the thinning and atrophy, and, in some cases, bleeding, of the center of the eye's lining within the macular region of the retina. This may lead to the loss of central vision, and as a result, patients cannot see more detailed content. According to a report by the American Academy of Ophthalmology, macular degeneration is a main cause of central vision loss (blindness) in old people. Although some macular dystrophies that affect young people are also called macular degeneration sometimes, this term generally refers to age-related macular degeneration (AMD). Age-related macular degeneration begins with characteristic yellow deposits (called drusen) located in the macula lutea (a retinal central region providing detailed central vision, called fovea) between the retinal pigment epithelium and the choroid thereunder. People with drusen will continue to develop advanced AMD. It is very dangerous if the drusen increases in size and number and is related to the disorder in the submacular pigmented cell layer.

There are two forms of advanced AMD that cause deep vision loss: dry and wet. Central atrophy, *i.e.,* the dry form of advanced AMD, which is resulted from the atrophy of the subretinal retinal pigment epithelium layer, causes vision loss by the loss of photoreceptors (rods and cones) at the center of the eye.

Age-related macular degeneration is a disease of irreversible vision decrease or loss caused by the degeneration of retinal pigment epithelial cells and the neural retina. It is most common in patients over 50 years old, occurs in both eyes successively or simultaneously and impairs vision progressively, so it is a fundus lesion that severely threatens the visual function of old people. With the aging of population, age-related macular degeneration has become the leading cause of blindness in western countries, and its incidence in Asia is also gradually on the increase.

The development of modern biotechnology enables us to produce anti-VEGF monoclonal antibodies through recombinant DNA technology, and this technology has been widely applied in the production of a variety of monoclonal antibodies. Residues from rodent antibodies can be selected to substitute some sites in CDR regions or framework regions in a human antibody to form a humanized monoclonal antibody, thus decreasing antigenicity. So far, a variety of humanized anti-VEGF monoclonal antibodies have been successfully produced, which show significant affinity to hVEGF and inhibitory activity *in vivo* and *in vitro.* For example, a specific humanized anti-VEGF antibody Bevacizumab has been used in clinical trials for treating solid tumors, and other humanized anti-VEGF antibodies Ranibizumab and Aflibercept have treated age-related macular degeneration associated with choroid neovascularization with high affinity. Before a therapeutic antibody is used in the human body, preclinical studies need to be carried out in a non-human mammal to evaluate the effectiveness and toxicity of the monoclonal antibody used. Ideally, the antibody that has been subjected to these preclinical studies can identify and react with a target antigen of the host animal (mouse, rabbit or non-human primate) at high efficiency.

Bevacizumab (Avastin), another humanized IgG1 anti-VEGF monoclonal antibody developed by Genentech, is a human anti-tumor monoclonal antibody that is produced in Chinese hamster ovary cells (CHOs) as a mammalian cell expression system by adopting recombinant DNA technology and then purified by adopting processes including viral inactivation and removal.

Ranibizumab (Lucentis), a humanized monoclonal antibody Fab fragment developed by Genentech and produced in escherichia coli by recombinant DNA technology, targets VEGF-A and can bind to various subtypes of VEGF-A with high affinity, such as $VEGF-A_{121}$, $VEGF-A_{165}$ and $VEGF-A_{110}$. Ranibizumab binds to VEGF-A to prevent VEGF-A from binding to its receptors (VEGFR-1 and VEGFR-2) located on the surface of endothelial cells, thus preventing vascular endothelial hyperplasia, reducing vascular leakage and inhibiting choroidal neovascularization (CNV).

Both Ranibizumab and Bevacizumab are derived from the same murine parent antibody A4.6.1. A4.6.1 is a mouse anti-VEGF monoclonal antibody that is produced with a hybridoma cell line derived from human $VEGF-A_{165}$ protein-immunized mice. In order to decrease immunogenicity that is generated when a human disease is treated by the murine antibody, the gene of antibody A4.6.1 has been humanized by recombinant DNA technology. The humanized Fab fragment keeps the murine CDR sequences, and is then combined with a human Fc fragment to obtain a full-length antibody, *i.e.* Bevacizumab. Bevacizumab has the same affinity for antigen as its parent antibody, but has lower immunogenicity and longer *in vivo* half-life.

Ranibizumab is a high-affinity antibody that is obtained by site-directed mutagenesis and screening of amino acids on the basis of the Fab region of Bevacizumab. The Fab regions of Ranibizumab and Bevacizumab have six different amino acids in total. The mutagenesis of these six amino acids increase the affinity of Ranibizumab for VEGF-A by five to twenty times in comparison with that of Bevacizumab. An *in vitro* experiment proved that the strength of Ranibizumab in inhibiting HUVEC proliferation is 5.2 times higher than that of Bevacizumab. Ranibizumab, which is a Fab fragment with a small molecular weight, can fully permeate all the layers of the retina in 1 h and has an intravitreal half-life of 3.2 days, while Bevacizumab cannot permeate the internal limiting membrane of the monkey retina. In addition, systemic adverse reaction to Ranibizumab is minor since Ranibizumab is a small antigen-binding fragment without Fc region, does not trigger complement-mediated immunoreaction and has a short systemic half-life.

For eye diseases, a small antibody fragment (such as Fab or $(Fab)_2$) applied in the vitreous body is usually used because of its short serum half-life and low risk of systemic toxicity. However, such a small antibody fragment will lead to a short intravitreal half-life (due to rapid diffusion into serum), and must be administered more frequently, causing mental and physical burden on patients during treatment. Therefore, antibodies that have long half-lives and comprise Fc domains have also been applied in the treatment of eye diseases. For example, Aflibercept produced by Bayer is an Fc fusion protein.

## SUMMARY OF THE INVENTION

The objective of the present invention is to provide an anti-VEGF antibody with high affinity. The objective of the present invention is also to provide an anti-VEGF antibody with a long *in vivo* half-life.

The objective of the present invention is also to provide an anti-VEGF antibody with lower immunogenicity.

The antibody of the present invention is a specially designed full-length humanized anti-VEGF IgG1 antibody for treating fundus lesions. The antibody of the present invention has affinity for antigen similar to that of Ranibizumab. However, the antibody of the present invention is characterized by absence of CDC and ADCC effects, higher safety in intravitreal injection, longer half-life in the vitreous body after being injected therein and better expected therapeutic effect. In one aspect, the present invention provides a full-length humanized anti-VEGF antibody, which has: (a) two immunoglobulin light chains; and (b) two immunoglobulin heavy chains; the heavy chains comprise a variable region and a constant region from the N-terminus to the C-terminus; and the antibody specifically recognizes the antigen-binding site of VEGF.

Still further, the heavy chains comprise an amino acid sequence having at least 80%, at least 85% or at least 90% identity with an amino acid sequence set forth in SEQ ID NO: 1; preferably, the heavy chains comprise an amino acid sequence having at least 91%, at least 92%, at least 93%, at least 94%, at least 95%, at least 96%, at least 97% or at least 98% identity with an amino acid sequence set forth in SEQ ID NO: 1; more preferably, the heavy chains comprise an amino acid sequence having at least 98.1%, at least 98.2%, at least 98.3%, at least 98.4%, at least 98.5%, at least 98.6%, at least 98.7%, at least 98.8% or at least 98.9% identity with an amino acid sequence set forth in SEQ ID NO: 1; further more preferably, the heavy chains comprise an amino acid sequence having at least 99%, at least 99.1%, at least 99.2%, at least 99.3%, at least 99.4%, at least 99.5%, at least 99.6%, at least 99.7%, at least 99.8% or at least 99.9% identity with an amino acid sequence set forth in SEQ ID NO: 1.

The light chains comprise an amino acid sequence having at least 80%, at least 85% or at least 90% identity with an amino acid sequence set forth in SEQ ID NO: 2; preferably, the light chains comprise an amino acid sequence having at least 91%, at least 92%, at least 93%, at least 94%, at least 95%, at least 96%, at least 97% or at least 98% identity with an amino acid sequence set forth in SEQ ID NO: 2; more preferably, the light chains comprise an amino acid sequence having at least 98.1%, at least 98.2%, at least 98.3%, at least 98.4%, at least 98.5%, at least 98.6%, at least 98.7%, at least 98.8% or at least 98.9% identity with an amino acid sequence set forth in SEQ ID NO: 2; further more preferably, the light chains comprise an amino acid sequence having at least 99%, at least 99.1%, at least 99.2%, at least 99.3%, at least 99.4%, at least 99.5%, at least 99.6%, at least 99.7%, at least 99.8% or at least 99.9% identity with an amino acid sequence set forth in SEQ ID NO: 2.

In some embodiments, the heavy chains comprise an amino acid sequence set forth in SEQ ID NO: 1, and the light chains comprise an amino acid sequence set forth in SEQ ID NO: 2.

In some embodiments, the antibody of the present invention is a monoclonal antibody, and further, the antibody is human IgG1.

In some embodiments, the antibody provided by the present invention can bind to human VEGF with a KD value approximate to that of Bevacizumab and inhibit the binding of VEGF to a VEGF receptor. More specifically, the antibody provided by the present invention can bind to human VEGF with a KD value of 10 nM or lower at 30 °C and inhibit the binding of VEGF to a VEGF receptor. According to another embodiment, the KD value is 1 nM or lower. According to another embodiment, the antibody of the present invention can bind to VEGF with a KD value of no more than about 500 pM.

According to one embodiment, the antibody of the present invention binds to human and/or mouse VEGF with an on rate ($k_{on}$) of 1.0 or higher (10 M$^{-1}$ S$^{-1}$). According to another embodiment, this on rate is 4.0 or higher (10 M$^{-1}$ S$^{-1}$).

According to another embodiment, the antibody of the present invention, with an ideal affinity, binds to human VEGF, further to VEGF-A, and still further to VEGF-A165, but does not bind to any or all VEGF-related homologues composed of human VEGF-B, human VEGF-C or human VEGF-D.

The VEGF receptor inhibited from binding to VEGF may be VEGF receptor 1 (Flt-1), VEGF receptor 2 (Flk-1) or both.

According to one embodiment, the monoclonal antibody of the present invention contacts the 20s helix of VEGF. According to another embodiment, the monoclonal antibody of the present invention contacts the 80s loop of VEGF. According to another embodiment, the monoclonal antibody of the present invention contacts the 20s helix and 80s loop of human VEGF.

Another aspect of the present invention provides a nucleic acid encoding the heavy chains and/or light chains of the antibody of the present invention.

The present invention further provides an expression vector comprising the nucleic acid of the present invention, and the expression vector can express the nucleic acid in a prokaryotic or eukaryotic host cell.

The present invention further provides a host cell comprising the vector, and the host cell is used to produce the antibody of the present invention.

The host cell is eukaryotic or prokaryotic.

The present invention further comprises a method for producing the antibody, which is characterized in that the nucleic acid of the present invention is expressed in the prokaryotic or eukaryotic host cell, and the antibody is isolated from the host cell or a host cell culture.

One embodiment comprises the following steps:

(a) the vector comprising the nucleic acid molecule encoding the antibody is used to transform the host cell;

(b) the host cell is cultured under a condition allowing the synthesis of the antibody molecule; and

(c) the antibody molecule is isolated from the culture.

The present invention further comprises an antibody obtained by the method for producing the full-length antibody.

The present invention further provides an affinity purification reagent. The affinity purification reagent comprises the aforementioned antibody, and the aforementioned antibody can be used to prepare a reagent capable of being used in the diagnostic analysis of VEGF protein. The antibody can be labeled with a detecting molecule. For example, the antibody can be labeled with a radioisotope, fluorescent label or enzyme.

The present invention further provides a kit for diagnosis of VEGF protein, and the kit comprises the aforementioned antibody or polypeptide.

The present invention further provides a pharmaceutical preparation comprising the antibody. The pharmaceutical preparation further comprises a pharmaceutically acceptable carrier.

The present invention also provides a use of the antibody or polypeptide in the preparation of a medicament for treating a disease associated with VEGF overexpression in a mammal.

Treating the mammal described herein comprises administering an effective amount of the antibody to the mammal. The dosage of the antibody will be the effective amount for treating the disease. In a study on increased doses, multiple doses of the antibody can be administered to the mammal. In another embodiment, a therapeutically effective amount of the antibody is administered to a human patient to treat a disease.

The mammal may be a human or a non-human mammal, such as a primate or a rodent (such as mouse, rat or rabbit) suitable for generating preclinical data. Preferably, the mammal is human being.

The mammal may be healthy or suffer from a disease that needs to be treated with the antibody. In the present invention, the disease associated with VEGF overexpression or the disease is a disease related to abnormal angiogenesis caused by VEGF overexpression, and further, a fundus lesion caused by VEGF overexpression.

As a preferred embodiment, the antibody of the present invention is used to treat wet (neovascular) age-related macular degeneration (wet-AMD), choroidal neovascularization (CNV) secondary to pathological myopia (PM), diabetic macular edema (DME), diabetic retinopathy (DR), branch retinal vein occlusion (BRVO), central retinal vein occlusion (CRVO) and other fundus lesions. The antibodies can be used in a rhesus monkey model having laser-induced choroidal neovascularization. The antibodies can be used to identify a method of treating age-related macular degeneration with the anti-VEGF antibody of the present invention by observing or monitoring the area change of macular degeneration treated with the anti-VEGF antibody of the present invention. The antibody of the present invention can also be used in the research and evaluation of combination therapies adopting the anti-VEGF antibody of the present invention and other therapeutics. The antibody of the present invention can be used to study the effect of VEGF in other diseases by administering the antibody or polypeptide to animals suffering from similar diseases and determining whether one or more symptoms of the diseases are relieved.

In some aspects, the present invention further provides an antibody derivative. The antibody of the present invention can be further modified to comprise other non-protein portions well-known in the art, such as water-soluble polymers.

In some aspects, the present invention further provides an immunoconjugate (antibody-drug conjugate or ADC), which comprises an antibody in which the antibody of the present invention is conjugated with one or more cytotoxic reagents, and the cytotoxic reagent may be, for example, chemotherapeutic agent, drug, production inhibitor, toxin or radioisotope.

The antibody of the present invention is more beneficial to patients with retinal macular degeneration disease due to the existence of its Fc portion. Compared with a small antibody fragment without constant heavy chain regions, the antibody of the present invention is highly stable and diffuses slowly from the vitreous body in the intravitreal environment, *i.e.,* the half-life is prolonged, and thus the drug action time is prolonged, wherein the actual disease is located and treated here. Therefore, compared with non-IgG-like antibodies, such as Fab and (Fab)$_2$ fragments, the antibody of the present invention can prolong the treatment cycle and improve the physiological and psychological conditions of patients.

The antibody of the present invention is a full-length humanized antibody. Compared with murine antibodies and chimeric antibodies, the full-length humanized antibody has multiple potential advantages in safety and efficacy. Compared with other types of antibodies, the full-length humanized antibody typically shows a low clearance rate. The low clearance rate can allow low dosage and frequency of administration.

According to one preferred embodiment, the antibody of the present invention is synthesized by a recombination method rather than directly produced from a hybridoma or derived from an antibody sequence from a hybridoma. In one preferred embodiment, the antibody binds to hVEGF-A$_{165}$ with a KD value of no more than about 2 nM, about 1 nM or about 500 pM. The full-length antibody of the present invention shows good efficacy in inhibiting fundus diseases, particularly choroidal neovascularization.

**BRIEF DESCRIPTION OF THE DRAWINGS**

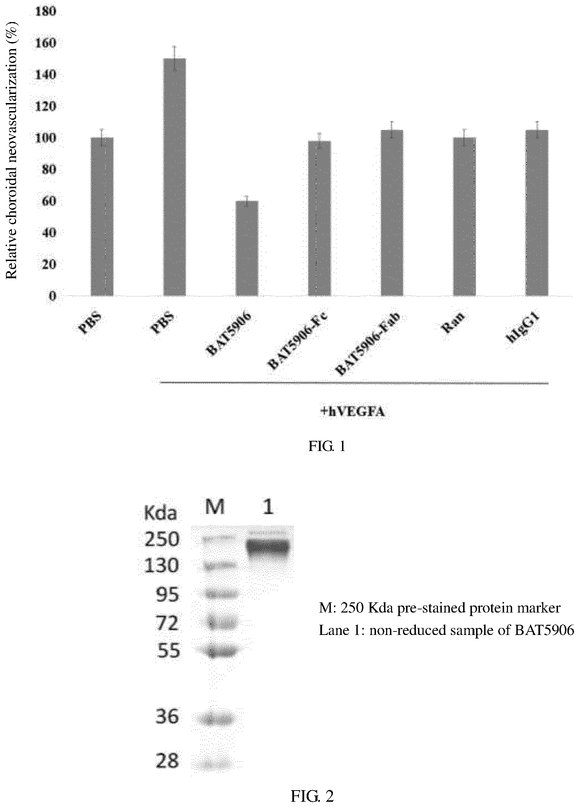

FIG. 1 depicts an experiment comparing the neovascularization-inhibiting effect of different structures of BAT5906 and Ranibizumab, showing that the neovascularization-inhibiting effect of the complete molecule of the monoclonal antibody BAT5906 is better than that of the commercially available drug Ranibizumab.

FIG. 2 shows an SDS-PAGE (non-reduced) result of the monoclonal antibody BAT5906 (M: 250 kDa pre-stained protein marker; Lane 1: non-reduced sample of BAT5906).

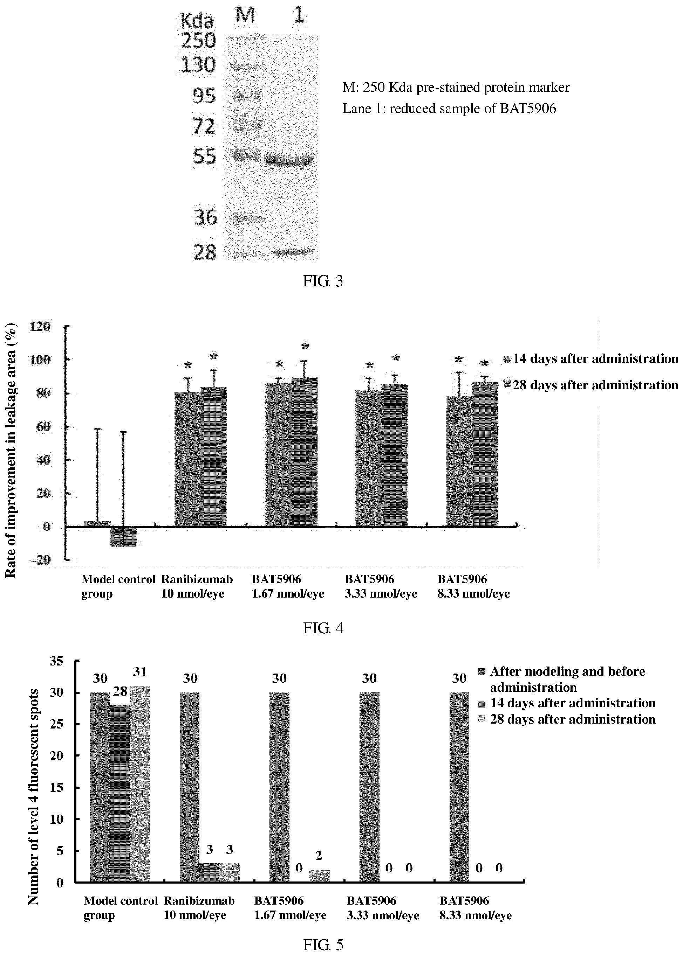

FIG. 3 shows an SDS-PAGE (reduced) result of the monoclonal antibody BAT5906 (M: 250 kDa pre-stained protein marker; Lane 1: reduced sample of BAT5906). The results in FIG. 2 and FIG. 3 show that the size of the monoclonal antibody molecule is equal to a designed size in both reduced and non-reduced conditions.

FIG. 4 depicts the rate (%) of improvement in fluorescein leakage areas of fundus laser spots of each group of monkeys after administration, showing that the monoclonal antibody BAT5906 has effectively inhibited the fluorescein leakage area in a pharmacodynamic experiment on rhesus monkeys, with the commercially available drug Ranibizumab as a control. Compared with a model control group, the difference of means is statistically significant (P ≤ 0.05).

FIG. 5 depicts the number of level 4 fluorescent spots of eyeballs of each group of monkeys before and after administration, showing that the monoclonal antibody BAT5906 has effectively inhibited the number of level 4 fluorescent spots in a pharmacodynamic experiment on rhesus monkeys, with the commercially available drug Ranibizumab as a positive control.

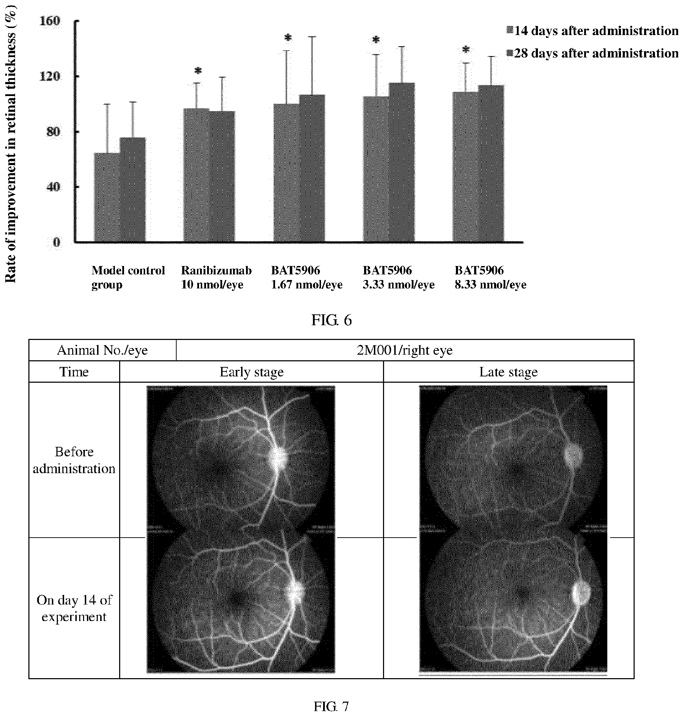

FIG. 6 depicts the rate (%) of improvement in retinal thicknesses at sites with the severest retinal lesion of each group of monkeys after administration, showing that the monoclonal antibody BAT5906 has effectively inhibited the thickening of the retina of the fundus in a pharmacodynamic experiment on monkeys, with the commercially available drug Ranibizumab as a positive control. Compared with a model control group, the difference of means is statistically significant (P ≤ 0.05).

FIG. 7 provides fluorescein angiograms of a monkey eyeball in an acute toxicity experiment on rhesus monkeys and the detection was conducted once before administration and once 14 days after intravitreal injection. Fundus fluorescein angiography test shows that retinal vessels are evenly perfused in the early stage (within about 1 min) and the late stage (about 5 min later) after angiography, arterial filling time and venous filling time are normal, and retinal venous dilation and effusion, retinal non-perfusion areas, blocked fluorescence, neovascularization and other manifestations are not found, indicating that the antibody of the present invention is highly safe in intravitreal injection.

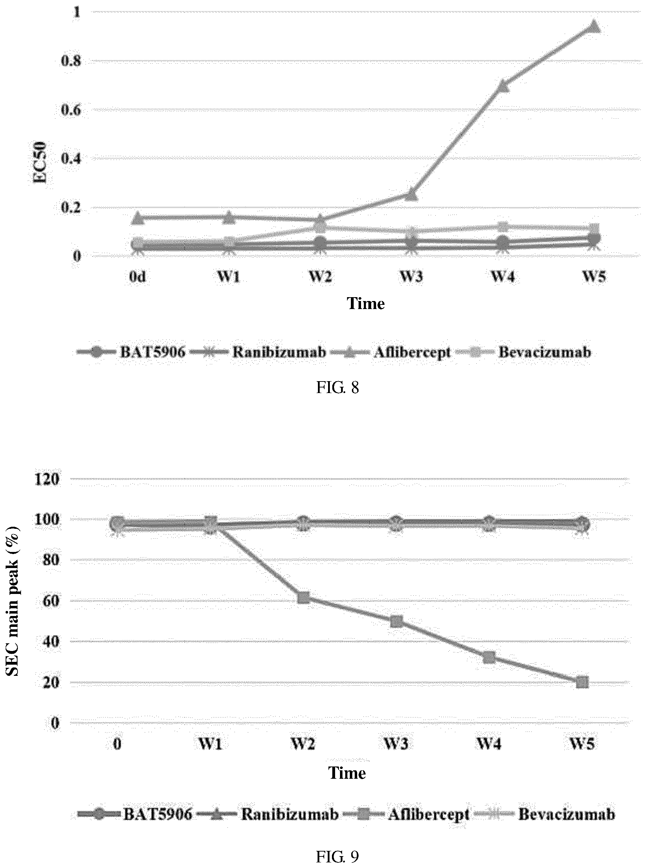

FIG. 8 depicts the changes of cytobiological activities at 37 °C after BAT5906 and control ophthalmological drugs are diluted, showing that the monoclonal antibody BAT5906 still keeps a stable cytobiological activity under the condition of simulating the concentration of injection in the human vitreous body and can inhibit VEGF at the site of fundus lesion.

FIG. 9 depicts the changes of SEC main peaks at 37 °C after BAT5906 and control ophthalmological drugs are diluted, showing that the monoclonal antibody BAT5906 still keeps a stable structure under the condition of simulating the concentration of injection in the human vitreous body and can inhibit VEGF at the site of fundus lesion.

**DETAILED DESCRIPTION OF THE INVENTION**

The technical solution of the present invention will be further illustrated below through specific examples, which are not intended to limit the protection scope of the present invention. Some nonessential modifications and adjustments made by other people according to the concept of the present invention still fall within the protection scope of the present invention.

**I. Definitions**

Unless otherwise indicated, the descriptions and requirements involved herein are defined as follows.

It should be noted that unless otherwise indicated, a singular form described herein includes a plural meaning. For example, when a "complex" is mentioned, the plural form of the complex is also included.

"About" described herein should be understood by those of ordinary skill in the art, and its application can be broadened in a simple way. If the application of any term is unclear to those of ordinary skill in the art, the context of the application should be provided. "About" refers to +10% to -10%, +5% to -5% or +1% to -1% of a specific value.

The term "comprise" described herein means that a composition and method comprise the substances without excluding other substances. When "mainly comprise" is used to define a composition and method, it should mean that any other important component is excluded. For example, a complex comprises the important components but excludes other important components. For example, if a complex consists of the defined important components, those components that do not have significant influence on the basic properties and novelty of the present invention are not excluded. "Consist of" means excluding ingredients exceeding a trace amount and important methods and steps. Embodiments defined by these terms are limited within the scope of the present invention.

As used herein, "antibody (Ab)" or "antigen-binding unit (Abu)" refers to a protein or molecule comprising one or more antigen-binding sites. This term includes, but is not limited to, full-length antibodies and antibody fragments. In a certain aspect, an antibody comprises heavy chain variable regions (VHs) and/or light chain variable regions (VLs) or a pair of VH/VL, and may be a full-length antibody or antibody fragment, such as a single-chain Fv or VH domain and/or VL domain, a Fab or a (Fab)$_2$. In a certain aspect, each antigen-binding site comprises an antibody heavy chain variable region (VH) and/or an antibody light chain variable region (VL), or may consist of a pair of antibody light chain variable region (VL) polypeptide and antibody heavy chain variable region (VH) polypeptide.

As used herein, the term "recombinant humanized antibody" refers to all humanized antibodies prepared, expressed, created or isolated by employing recombination methods, such as antibodies isolated from NSO or CHO host cells, antibodies obtained from transgenic animals (such as mouse) expressing human immunoglobulin gene or antibodies transfected into host cells for expression using recombinant expression vectors.

The term "hypervariable region" or "the antigen-binding portion of an antibody" refers to amino acid residues of the antibody responsible for antigen binding when used herein. The hypervariable regions include "complementarity determining regions" or "CDR" amino acid residues. "Framework" or "FR" regions are the regions of those variable domains other than the hypervariable region residues defined herein. Therefore, the light chains and heavy chains of an antibody comprise domains FR1, CDR1, FR2, CDR2, FR3, CDR3 and FR4 from the N-terminus to the C-terminus. Such framework amino acids separate the CDRs of each chain. The CDRs of each chain are separated by such framework amino acids. In particular, the heavy chain CDR3 is a region mainly responsible for antigen binding. The determination of the CDR and FR regions is based on the standard definition of Kabat, *et al.* (Kabat, et al., Sequences of Proteins of Immunological Interest, 5th ed., Public Health Service, 15 National Institutes of Health, Bethesda, Md. (1991)).

When used herein, the expressions "cell", "cell line" and "cell culture" can be used interchangeably, and all these designations include their progenies. Therefore, the words "transformant" and "transformed cell" include primary test cells and cultures derived therefrom, regardless of the number of transfers. It should also be understood that the DNA contents of all progenies may not be precisely consistent due to intentional or accidental mutations. Variant progenies having the same function or biological activity that are screened from the initially transformed cells are included. When referring to different designations, they will become clear through the context.

When used herein, the term "transform" refers to a process of transferring a vector/nucleic acid into a host cell. If a cell without an insurmountable cell wall barrier is used as a host cell, transfection is carried out by, for example, the calcium phosphate precipitation method described in Graham, F.L., van der Eb, A.J., Virology, 52(1973)546-467. However, other methods for introducing DNA into cells can also be used, such as nuclear injection or protoplast fusion. If a prokaryotic cell or a cell comprising a parenchyma cell wall structure is used, for example, one transfection method is calcium treatment with calcium chloride, as described in Cohen, S.N., et al., PNAS (Proceedings of the National Academy of Sciences).69(1972)2110-2114.

When used herein, "expression" refers to a process of transcribing a nucleic acid into mRNA and/or a process of subsequently translating the transcribed mRNA (also called transcript) into a peptide, a polypeptide or a protein. Transcripts and encoded polypeptides are collectively referred to as gene products. If a polynucleotide is derived from genomic DNA, expression in eukaryotic cells may include mRNA splicing.

A "vector" is a nucleic acid molecule, particularly a self-replicating one, which transfers an inserted nucleic acid molecule into a host cell and/or between host cells. This term includes vectors with a main function of inserting DNA or RNA into cells (such as chromosomal integration), replicating vectors with a main function of replicating DNA or RNA and expression vectors with a function of transcribing and/or translating DNA or RNA. Vectors that provide more than one of the aforementioned functions are also included.

An "expression vector" is a polynucleotide, which can be transcribed and translated into a polypeptide when introduced into a suitable host cell. An "expression system" typically refers to a suitable host cell comprising an expression vector, and the expression vector can function to produce a desired expression product.

The following examples, sequence tables and accompanying drawings are provided to assist in the understanding of the present invention, and the true objective is presented in the appended claims. It should be understood that modifications can be made to the method without departing from the spirit of the present invention.

The "treatment" of a patient's disease means: (1) preventing a disease from occurring in a patient who is prone to the disease or has not shown symptoms of the disease; (2) inhibiting the disease or preventing its development; or (3) relieving the disease or making it regress.

An "effective amount" refers to the amount of an active compound or medicament that results in a biological or drug response of tissues, systems, animals, individuals and humans which is being sought by researchers, vets, doctors or other clinical doctors, including the treatment of a disease.

## II. Full-length Antibody

In one example, the present invention provides a novel full-length antibody, which has high affinity and a therapeutic effect on macular degeneration disease.

A "full-length antibody" or "complete antibody" refers to an antibody or antibody portion comprising at least two light chains and two heavy chains, and each heavy chain comprises at least one variable region (VH) and three constant regions (such as CHI, CH2 and CH3).

In some aspects, each light chain, the variable regions, the constant regions of IgG1 and the VH and VL regions of scFv are all humanized sequences, which can be arbitrarily modified.

In some aspects, each heavy chain in the full-length antibody comprises an amino acid sequence having at least 80%, at least 85% or at least 90% identity with an amino acid sequence set forth in SEQ ID NO: 1; preferably, the heavy chain comprises an amino acid sequence having at least 91%, at least 92%, at least 93%, at least 94%, at least 95%, at least 96%, at least 97% or at least 98% identity with an amino acid sequence set forth in SEQ ID NO: 1; more preferably, the heavy chain comprises an amino acid sequence having at least 98.1%, at least 98.2%, at least 98.3%, at least 98.4%, at least 98.5%, at least 98.6%, at least 98.7%, at least 98.8% or at least 98.9% identity with an amino acid sequence set forth in SEQ ID NO: 1; further more preferably, the heavy chain comprises an amino acid sequence having at least 99%, at least 99.1%, at least 99.2%, at least 99.3%, at least 99.4%, at least 99.5%, at least 99.6%, at least 99.7%, at least 99.8% or at least 99.9% identity with an amino acid sequence set forth in SEQ ID NO: 1.

In some aspects, each light chain in the full-length antibody comprises an amino acid sequence having at least 80%, at least 85% or at least 90% identity with an amino acid sequence set forth in SEQ ID NO: 2; preferably, the light chain comprises an amino acid sequence having at least 91%, at least 92%, at least 93%, at least 94%, at least 95%, at least 96%, at least 97% or at least 98% identity with an amino acid sequence set forth in SEQ ID NO: 2; more preferably, the light chain comprises an amino acid sequence having at least 98.1%, at least 98.2%, at least 98.3%, at least 98.4%, at least 98.5%, at least 98.6%, at least 98.7%, at least 98.8% or at least 98.9% identity with an amino acid sequence set forth in SEQ ID NO: 2; further more preferably, the light chain comprises an amino acid sequence having at least 99%, at least 99.1%, at least 99.2%, at least 99.3%, at least 99.4%, at least 99.5%, at least 99.6%, at least 99.7%, at least 99.8% or at least 99.9% identity with an amino acid sequence set forth in SEQ ID NO: 2.

In some aspects, each heavy chain in the full-length antibody comprises an amino acid sequence of SEQ ID NO: 1.

In some aspects, each light chain in the full-length antibody comprises an amino acid sequence of SEQ ID NO: 2.

Heavy chain amino acid sequence (SEQ ID NO: 1):

EVQLVESGGGLVQPGGSLRLSCAASGYDFTHYGMNWVRQAPGKGLEWVGWINTYTG

EPTYAADFKRRFTFSLDTSKSTAYLQMNSLRAEDTAVYYCAKYPYYYGTSHWYFDVWG

QGTLVTVSSASTKGPSVFPLAPSSKSTSGGTAALGCLVKDYFPEPVTVSWNSGALTSGVH

TFPAVLQSSGLYSLSSVVTVPSSSLGTQTYICNVNHKPSNTKVDKKVEPKSCDKTHTCPP

CPAPELLGGPSVFLFPPKPKDTLMISRTPEVTCVVVDVSHEDPEVKFNWYVDGVEVHNA

KTKPREEQYNSTYRVVSVLTVLHQDWLNGKEYKCKVSNKALPAPIEKTISKAKGQPREP

QVYTLPPSREEMTKNQVSLTCLVKGFYPSDIAVEWESNGQPENNYKTTPPVLDSDGSFFL

YSKLTVDKSRWQQGNVFSCSVMHEALHNHYTQKSLSLSPGK

Light chain amino acid sequence (SEQ ID NO: 2):

DIQLTQSPSSLSASVGDRVTITCSASQDISNYLNWYQQKPGKAPKVLIYFTSSLHSGVPSR

FSGSGSGTDFTLTISSLQPEDFATYYCQQYSTVPWTFGQGTKVEIKRTVAAPSVFIFPPSDE

QLKSGTASVVCLLNNFYPREAKVQWKVDNALQSGNSQESVTEQDSKDSTYSLSSTLTLS

KADYEKHKVYACEVTHQGLSSPVTKSFNRGEC

**III. Therapeutic Regimen**

The present invention provides a method for treating macular degeneration disease or related diseases, and the use of an effective amount of the full-length antibody as described herein is required in this method. In some aspects, this disease is wet (neovascular) age-related macular degeneration (wet-AMD), choroidal neovascularization (CNV) secondary to pathological myopia (PM), diabetic macular edema (DME), diabetic retinopathy (DR), branch retinal vein occlusion (BRVO), central retinal vein occlusion (CRVO) or other fundus lesions.

The following examples further elaborate on some aspects of the present invention and help scientists familiar with this technical field implement the present invention. These examples are in no way intended to limit the scope of the present invention.

**Example 1: Study on Inhibition Mechanism of Recombinant Humanized Anti-VEGF Antibody against Neovascularization**

Heavy chain amino acid sequence and light chain amino acid sequence of the recombinant humanized anti-VEGF monoclonal antibody (antibody BAT5906) are respectively set forth in SEQ ID NO: 1 and SEQ ID NO: 2.

Laser photocoagulation was performed on both eyes of mice to destroy the choroid structure, forming neovascularization models. After a human VEGFA antigen was injected into the model, choroidal neovascularization was enhanced. Then the recombinant humanized anti-VEGF monoclonal antibody BAT5906, Ranibizumab (Ran), the Fab domain of the antibody BAT5906 (BAT5906-Fab), the Fc domain of the antibody BAT5906 (BAT5906-Fc) and human IgG1 (hIgG1) were respectively injected to the models. Compared with a control group (PBS), the choroidal neovascularization was inhibited in all the models. It was inferred that two different neovascularization inhibition mechanisms were present. Among them, BAT5906-Fc and human IgG1 exhibited a target-independent inhibitory effect, Ranibizumab and BAT5906-Fab exhibited a VEGF target-dependent inhibitory effect, and BAT5906, as a complete molecule, exhibited the highest degree of inhibition and had both inhibitory effects. The experiment results are shown in FIG. 1.

BAT5906 can inhibit neovascularization through the inhibition of VEGF pathway resulted from the specific binding of Fab domain to VEGF, and through the binding of the Fc domain to FcgRI as well. Containing the Fc domain, human IgG1 can inhibit neovascularization through the FcgRI pathway even without a Fab domain specifically binding to VEGF to inhibit the VEGF pathway, showing that the FcgRI pathway is also a way to inhibit neovascularization. Ranibizumab only has the Fab domain specifically binding to VEGF and can only inhibit neovascularization by inhibiting the VEGF pathway. Compared with Ranibizumab, BAT5906 has both the Fab domain specifically binding to VEGF and the Fc domain and can inhibit the VEGF pathway and the FcgRI pathway, thus enjoying a better neovascularization-inhibiting effect.

**Example 2: Construction of Recombinant Plasmid and Stable Cell Strain of Recombinant Anti-VEGF Monoclonal Antibody (Antibody BAT5906)**

According to an amino acid sequence, a recombinant expression plasmid pBAT5906 was constructed, which contained GS cDNA elements and was used to synthesize a glutamine synthetase gene as an amplifiable selectable marker for a stable cell strain, so that stable cell strain screening could be carried out by adding a certain amount of L-methionine sulfoximine (MSX) into medium. The constructed recombinant expression plasmid pBAT5906 was subjected to an enzyme digestion validation with the restriction enzyme Pvu I/Not I, and the result was consistent with an expected design result, proving that the construction of the recombinant expression vector pBAT5906 was successful.

A host cell strain for antibody expression, which was a derived cell line of CHO-K1 cells, was suspended in CD-CHO medium for growth. The process of constructing a stable cell strain expressing the antibody BAT5906 was as follows: the host cells in the logarithmic phase were centrifuged and re-suspended in fresh CD-CHO medium (cell density: $1.43 \times 10^7$ cells/mL). 600 $\mu$L of the aforementioned cell suspension was taken to be well mixed with 40 $\mu$g of linearized plasmid, and then the mixture was added into an electroporation cuvette for electroporation. The parameters of a Bio-rad electroporator were set as: capacitance: 960 $\mu$FD; voltage: 300 V; electroporation time: 15-20 ms. The electroporated cells were immediately re-suspended in 500 mL of CD-CHO medium preheated to 37 °C. The cell suspension was dispensed into a 96-well plate at 100 µL/well. Two to three days later, 100 µL of screening medium was replenished. Two to three weeks later, the concentration of antibody in the cell culture supernatant in the 96-well plate was determined, and the clones with a high expression level were transferred into a 24-well plate. When the cells grew to a certain number, the cells with a high antibody expression were then transferred into a 6-well plate. Finally, 20 to 30 high-expression cell strains were kept and transferred into shake flasks for further evaluation. The yield of the ultimately obtained full-length monoclonal antibody expression cell strain could reach about 3 g/L.

**Example 3: Expression and Purification of Monoclonal Antibody**

The process of expressing and purifying the monoclonal antibody was as follows: after cells were cultured on a large scale for 2 weeks, the cells were isolated from the medium by low-speed centrifugation, and the harvested supernatant was further centrifuged at high speed to obtain clear supernatant. The recombinant antibody was purified by a two-step method including affinity chromatography (Protein A) and ion exchange. The media used in purification were Mab Select SuRe LX produced by GE, Giga Cap Q-650M produced by TOSOH and POROS XS produced by ABI. The size correctness of the isolated and purified antibody was verified by the SDS-PAGE method (FIG. 2 and FIG. 3), and the results showed that the size of the BAT5906 band was correct under both reduced and non-reduced conditions.

**Example 4: Experiment on Effectiveness of Inhibition of Neovascularization in Eyes of Rhesus Monkeys**

A laser-induced monkey choroidal neovascularization model is an ideal model for wet-AMD. In order to effectively evaluate the efficacy of the antibody of the present invention and the commercially available drug Ranibizumab (Lucentis), laser was employed to cause choroidal neovascularization in rhesus monkeys, and an intravitreal drug injection experiment was then carried out to evaluate drug efficacy. After modeling was successful, the rhesus monkeys were grouped, four rhesus monkeys in each group. BAT5906 was administered by single binocular intravitreal injection at 0.25 mg/eye (1.67 nmol/eye), 0.5 mg/eye (3.33 nmol/eye) and 1.25 mg/eye (8.33 nmol/eye). 0.9% sodium chloride injection was administered to a model control group by intravitreal injection. In addition, a commercially available drug control group was set, with Ranibizumab injected in each eyeball at 0.5 mg/eye (molar dosage: 10 nmol/eye). All the aforementioned injection volumes were 50 µL. 28 days after administration, the rate of improvement in fluorescein leakage area, the rate of improvement in retinal thicknesses at the fundus, the VEGF content in aqueous humor and the number of level 3 and level 4 fluorescent spots at the fundus were determined. The results are shown as Table 1:

**Table 1. Pharmacodynamic evaluation of intravitreal injection of BAT5906 on rhesus monkeys**

| Index determination time | Model control group $\bar{x} \pm SD$ | Lucentis 0.5 mg/eye group $\bar{x} \pm SD$ | BAT5906 0.25 mg/eye group $\bar{x} \pm SD$ | BAT5906 0.5 mg/eye group $\bar{x} \pm SD$ | BAT5906 1.25 mg/eye group $\bar{x} \pm SD$ |
|---|---|---|---|---|---|
| Rate of improvement in fluorescein leakage area (%) | | | | | |
| 28 days after administration | -11.89 ± 68.70 | 83.69 ± 9.97* | 89.33 ± 9.97* | 85.28 + 5.15* | 86.46 ± 3.61* |
| Rate of improvement in retinal thickening at fundus (%) | | | | | |
| 28 days after administration | 75.91 ± 25.50 | 94.73 ± 24.66 | 106.61 ± 42 | 115.17 ± 26.33 | 113.45 ± 20.95 |
| VEGF content in aqueous humor (ng/mL) | | | | | |
| 29 days after administration | 76.470 ± 21.742 | 8.074 ± 2.683* | 5.133 ± 3.748* | 1.633 ± 0.909*▲ | 0.599 ± 0.129 |
| Number of fluorescent spots at the fundus (level 3 + level 4) | | | | | |
| 28 days after administration | 39 | 6* | 5* | 2* | 3* |

28 days after administration of the monoclonal antibody BAT5906 of the present invention to both eyes of the monkeys in the 0.25 mg/eye, 0.5 mg/eye and 1.25 mg/eye dose groups, the rates of improvement in fluorescein leakage area (the means of the rates of improvement were respectively 89.33 %, 85.28% and 86.46%) were all significantly higher than that in the model control group.

It can be seen from Table 1 above and FIG. 4 that the rates of improvement in fluorescein leakage area at the laser spots of the monkeys' fundus in the 0.25 mg/eye, 0.5 mg/eye and 1.25 mg/eye BAT5906 groups are slightly higher than that in 0.5 mg/eye Ranibizumab group (83.69%), and a low dose of BAT5906 has a better rate of improvement in fluorescein leakage area.

28 days after administration of the monoclonal antibody BAT5906 of the present invention to both eyes of the monkeys in the 0.25 mg/eye, 0.5 mg/eye and 1.25 mg/eye dose groups, the retinal thicknesses at the sites with the severest lesion of all the eyeballs were reduced to a certain extent, the pigment epithelia of part of the eyeballs were regular and continuous, and the retinal thicknesses of part of the eyeballs had approximated or reached and were even lower than the level prior to modeling. The rates of improvement and reduction in retinal thickness at the sites with the severest lesion in these BAT5906 groups (28 days after administration, the means of the rates of improvement were respectively 106.61 %, 115.17 % and 113.45 %) were all significantly higher than that in the model control group. It can be seen from Table 1 above and FIG. 6 that the rates of improvement in retinal thickening at the monkeys' fundus in the 0.25 mg/eye, 0.5 mg/eye and 1.25 mg/eye BAT5906 groups are slightly higher than that in the 0.5 mg/eye Ranibizumab group.

29 days after intravitreal injection of the antibody BAT5906 of the present invention at three doses, the VEGF concentrations in the aqueous humor of the rhesus monkeys in the three dose groups were all lower than those in the model control group and the positive control Ranibizumab group. This shows that the antibody of the present invention has a stronger inhibitory effect on VEGF in the ocular tissues than the positive control drug Ranibizumab.

It can be seen from Table 1 and FIG. 5 that in the comparison of changes in the number of the severest level 3 and level 4 fluorescent spots (significant fluorescein leakage, leakage beyond the edge of the spot), the numbers of level 3 and level 4 fluorescent spots 28 days after administration were both 6 in the 0.5 mg/eye Ranibizumab group, significantly reduced in comparison with those prior to administration (39), while the number of the level 3 and level 4 fluorescent spots in the 0.25 mg/eye BAT5906 group was 5 and the numbers of the level 3 and level 4 fluorescent spots in the 0.5 mg/eye and 1.25 mg/eye BAT5906 groups were respectively 2 and 3, less than that in the positive control Ranibizumab group.

The aforementioned pharmacodynamic experiment result indicates that, compared with Ranibizumab, the monoclonal antibody BAT5906 of the present invention has a higher degree of inhibition of fluorescein leakage, and thus the efficacy is slightly better than that of Ranibizumab.

**Example 5: CDC Effect Detection Experiment and ADCC Effect Detection Experiment 1. CDC effect detection experiment**

Target cells (HUVEC cells) were re-suspended with complete endothelial cell medium (ECM) 24 h in advance to adjust the cell concentration to $2 \times 10^5$ cells/mL, and 50 $\mu$L of cell suspension was added into each well in a flat-bottom clear 96-well plate. Antibodies were diluted with DMEM/F12 medium containing 2% FBS and 5% complement (initial concentration of 40 $\mu$g/mL, diluted 1/5-fold in sequence, 3 replicate wells set for each concentration, eight gradients set in total). A pipette was used to thoroughly suck the medium in the aforementioned step, and the target cells diluted in the previous step were then added into a 96-well plate at 50 $\mu$L per well according to the experiment design, and then continued to be cultured in an incubator under the conditions of 37 °C and 5% $CO_2$ for at least 4 h. Antibodies at each concentration gradient were added into the 96-well plate each at 50 $\mu$L/well, with the final concentrations of the samples being 10 $\mu$g/mL, 2 $\mu$g/mL, 0.4 $\mu$g/mL, 0.08 $\mu$g/mL, 0.016 $\mu$g/mL, 0.0032 $\mu$g/mL, 0.00064 $\mu$g/mL and 0 $\mu$g/mL, and the samples were then cultured in the incubator under the conditions of 37 °C and 5% $CO_2$ for 30 min. Target cell maximum LDH release wells, target cell spontaneous LDH release wells, volume correction control wells and medium background wells were set (10$\mu$L of lysis buffer was added into the maximum LDH release wells and the volume correction control wells 45 min before detection), three replicate wells for each type of wells. The plate was incubated in the incubator under the conditions of 37 °C and 5% $CO_2$ for at least 4 h. Four hours later, the 96-well plate was centrifuged at 250 g for 4 min. A pipette was used to carefully suck the supernatant and transfer it into another 96-well plate, with 50 $\mu$L of LDH assay reagent added in each well. Incubation was performed in the dark under room temperature for 20-30 min, and 50 $\mu$L of stop solution was then added into each well. With 490 nm as the detection wavelength, absorbance was measured with a microplate reader. Calculation of experiment result: The mean of medium background absorbance was subtracted from the absorbance of all the experimental wells and the target cell spontaneous LDH release wells, and the mean of volume correction control absorbance was subtracted from the absorbance of the target cell maximum LDH release wells. The aforementioned corrected values were then substituted into the following formula to calculate percent cytotoxicity generated by each effector-to-target ratio.

$$\% \text{ cytotoxicity} = (\text{experimental} - \text{target cell spontaneous})/(\text{target cell maximum} - \text{target cell spontaneous}) \times 100\%$$

It can be seen from the results above that the Bevacizumab sample and the BAT5906 sample, under the same experiment conditions, do not have obvious difference in toxic effect on the target cells under the action of the complement and that the relative toxic effect on the target cells is about 0%, indicating that the antibody BAT5906 has no CDC effect. The results above indicate that the antibody of the present invention will not cause complement-dependent cytotoxicity in the cells (see Table 2).

## 2. ADCC effect detection experiment

Target cells (HUVEC cells) were re-suspended with 1640 medium 24 h in advance to adjust the cell concentration to $2 \times 10^5$ cells/mL, and 50 $\mu$L of cell suspension was added into each well in a flat-bottom clear 96-well plate.

Antibodies were diluted with DMEM/F12 medium containing 2% FBS (initial concentration of 40 $\mu$g/mL, diluted 1/5-fold in sequence, 3 replicate wells set for each concentration, eight gradients set in total).

Antibodies at each concentration gradient were added into sample wells each at 50 $\mu$L/well, with the final concentrations of the samples being 10 $\mu$g/mL, 2 $\mu$g/mL, 0.4 $\mu$g/mL, 0.08 $\mu$g/mL, 0.016 $\mu$g/mL, 0.0032 $\mu$g/mL, 0.00064 $\mu$g/mL and 0 $\mu$g/mL, and the samples were then cultured in an incubator under the conditions of 37 °C and 5% $CO_2$ for 30 min.

Effector cells PBMC in the logarithmic phase were taken and centrifuged at 800 rpm for 5 min to discard supernatant. DMEM/F12 medium containing 2% FBS was added. Then centrifugation at 800 rpm for 5 min was performed twice to discard supernatant and cell counting was also carried out, so as to adjust the effector cell concentration to $2 \times 10^5$ cells/mL.

A pipette was used to add the effector cells diluted in the previous step into the sample wells, 50 $\mu$L in each well, and the samples were then put into the incubator under the conditions of 37 °C and 5% $CO_2$ and continued to be cultured for at least 4 h.

Target cell maximum LDH release wells, target cell spontaneous LDH release wells, volume correction control wells and medium background wells were set (10 $\mu$L of lysis buffer in the detection kit was added into the maximum LDH release wells and the volume correction control wells 45 min before detection), three replicate wells for each type of wells. The plate was incubated in the incubator under the conditions of 37 °C and 5% $CO_2$ for at least 4 h.

After 4 h of culturing, the 96-well plate was centrifuged at 250 g for 4 min. A pipette was used to carefully suck the supernatant and transfer it into another 96-well plate, with 50 $\mu$L of LDH assay reagent added in each well. Incubation was performed in the dark under room temperature for 20-30 min, and 50 $\mu$L of stop solution was then added into each well. With 490 nm as the detection wavelength, absorbance was measured with a microplate reader.

It can be seen from the experiment results above that the antibody BAT5906 of the present invention and Bevacizumab, under the same experiment conditions, do not have obvious difference in toxic effect on the target cells under the action of the antibodies and that the relative toxic effect on the target cells is about 0%, indicating that the antibody BAT5906 has no ADCC effect (see Table 2).

The CDC and ADCC experiment results indicate that the antibody of the present invention will not cause *in vivo* complement-dependent cytotoxicity (CDC) and antibody-dependent cytotoxicity (ADCC), and will not affect the normal function of the immune system when used *in vivo*. As a low-toxicity anti-VEGF monoclonal antibody, the antibody of the present invention can normally exert its anti-VEGF function at a focus without causing adverse toxic reaction, and is highly safe when used in the human body.

### Table 2. CDC effect and ADCC effect experiment results of BAT5906 and control

| Category | CDC effect | ADCC effect |
| --- | --- | --- |
| Buffer | - | - |
| BAT5906 | - | - |
| Bevacizumab | - | - |
| Rituximab | - | + |
| * - represents the absence of such effect, and + represents the presence of such effect | | |

## Example 6: Acute Toxicity Experiment of Intravitreal Injection of Monoclonal Antibody in Rhesus Monkeys' Eyes

Two groups were set in this experiment, *i.e.,* a negative control group and a 8.0 mg/eye recombinant humanized anti-VEGF monoclonal antibody (BAT5906) injection group, three rhesus monkeys (female and male) in each group. A negative control (0.9% sodium chloride injection) and 80 mg/mL of recombinant humanized anti-VEGF monoclonal antibody injection were administered to the groups of rhesus monkeys respectively at a volume of 100 $\mu$L/eye by single

binocular intravitreal injection. The day of administration was defined as the first day of the experiment.

After administration, the general condition of each group of monkeys was observed every day for 14 days on end; on days 8 and 14 of the experiment, body weights were measured; on days 1 (before administration), 2, 4, 8 and 14 of the experiment, indirect ophthalmoscopy and slit lamp examination were performed; before administration, about 10 to 15 min, 1 h and 24 h after administration and on day 14 of the experiment, intraocular pressure was measured; before administration and on day 14 of the experiment, fundus photochromy and fluorescein angiography were performed; on days 2 and 14 of the experiment, hematological examination and blood biochemical examination were performed; and on day 15 of the experiment, all the rhesus monkeys were euthanized by bloodletting under anesthesia, and then gross anatomical observation was performed.

The experiment results show that under these experiment conditions, ocular toxicity and systemic toxicity were not discovered after 8.0 mg/eye of recombinant humanized anti-VEGF monoclonal antibody injection was administered to the rhesus monkeys by single binocular intravitreal injection and observation was performed for 14 days, and the maximum tolerated dose (MTD) was 8.0 mg/eye. Fundus fluorescein angiography in the acute toxicity experiment is shown in FIG. 7.

**Example 7: Experiment on Ocular Tissue Distribution of Intravitreally Injected Monoclonal Antibody in Rhesus Monkeys' Eyes**

In this experiment, 8.33 nmol/eye (*i.e.,* 1.25 mg/eye) intravitreal injection group was set, and 21 rhesus monkeys (10 females and 11 males) were used. 25 mg/mL of recombinant humanized anti-VEGF monoclonal antibody (BAT5906) injection was administered to both eyes of the monkeys in the intravitreal injection group at a volume of 50 μL/eye by single intravitreal injection. 4 h, 10 h, 24 h, 72 h, 168 h, 336 h and 672 h after administration, three monkeys each from the intravitreal injection group were euthanized (anesthesia with pentobarbital sodium at 30 mg/kg and bloodletting through the femoral artery), and anatomy was then performed to take out the eyeballs and optic nerves of both eyes and separate the aqueous humor, corneas, irises, vitreous bodies, lenses, retinas, choroids and optic nerves of the eyeballs. Before administration and 30 min, 1 h, 2 h, 4 h, 10 h, 24 h, 48 h, 72 h, 96 h, 168 h, 336 h and 672 h after administration, blood was collected from six monkeys in the intravitreal injection group that were anatomized 336 and 672 h after administration, and the serums were separated. The ELISA method was adopted to detect the concentration of the drug in each ocular tissue and serum, and the parameters of drug metabolism in the serums and the ocular tissues were calculated.

The main results are as follows:
After the recombinant humanized anti-VEGF monoclonal antibody injection was administered to the rhesus monkeys at 1.25 mg/eye (8.33 nmol/eye) by single binocular intravitreal injection, the drug could be rapidly diffused and distributed in each ocular tissue, the drug could be detected in each ocular tissue 4 h after injection, and the drug concentration in all the ocular tissues except for the corneas and the optic nerves reached a peak 4 to 24 h after administration. The descending order of the exposure levels ($AUC_{last}$) of the drug in all the ocular tissues was as follows: vitreous body, retina, cornea, aqueous humor, choroid, iris, lens and optic nerve. The elimination half-life in the main ocular tissues (such as vitreous body, aqueous humor, retina, choroid, iris and lens) was between 73.74-143.81 h, and the half-life in the vitreous body was 84.2 h.

The comparison between two anti-VEGF therapeutics and BAT5906 is shown in Table 3. The half-life of BAT5906 is significantly better than that of the two anti-VEGF therapeutics in the current market.

**Table 3. Comparison between properties of two anti-VEGF therapeutics and BAT5906**

| Category | Ranibizumab | Aflibercept | BAT5906 |
|---|---|---|---|
| Molecular structure | Fab | VEGFR-Fc | IgG1 |
| Molecular weight | 48 kD | 115 kD | 149 kD |
| Half-life in monkey's vitreous body | 55.7 h[a] | 40-60 h[b] | 84.2 h |

[a]Gaudreault J, Fei D, Rusit J, et al. Preclinical pharmacokinetics of Ranibizumab (rhuFabV2) after a single intravitreal administration[J]. Investigative ophthalmology & visual science, 2005, 46(2): 726-733.
[b] Biologic License Application (BLA):125387 Orig1s000 PHARMACOLOGY REVIEW(S), Company: REGENERON PHARMACEUTICALS, 11/18/2011.

It can be seen from Table 3 that after the monoclonal antibody of the present invention was administered to the rhesus monkeys at 8.33 nmol/eye by binocular intravitreal injection, the main parameter of drug metabolism in the monkey's vitreous body, *i.e.,* elimination half-life ($t_{1/2}$), was 84.2 h, whereas the half-life of the commercially available positive control drug Ranibizumab in the monkey's vitreous body subsequent to intravitreal injection was 55.7 h, and the half-life of

Aflibercept in the monkey's vitreous body subsequent to intravitreal injection was 40-60 h. Compared with the two commercially available positive control drugs mentioned above, the monoclonal antibody of the present invention has a longer half-life in the vitreous body, and therefore can exert efficacy in the eye for a longer time, exhibiting better effect in inhibiting fundus neovascularization.

## Example 8: Detection with Differential Scanning Calorimetry (DSC)

In order to study the thermal transition temperature of the antibody of the present invention, a capillary differential scanning calorimeter was used to detect the thermal unfolding temperature of each domain of the BAT5906 molecule. The instrument model was Nano DSC, the volumes of both the capillary sample cell and the reference cell were 0.300 mL, the heating rate was 1 °C/min, the pre-equilibrium time was 10 min, and the filtration period was 10 s. It can be seen from the experiment results in Table 4 that the BAT5906 molecule has two melting peaks; the first peak is a CH2 melting peak, with a Tm value being 76.07 °C; and the second peak is a Fab/CH3 melting peak, with a Tm value being 83.68 °C. The data above indicate that the overall structure of the BAT5906 molecule is relatively stable and the minimum Tm value in the domains also reaches 76.07 °C. The thermal unfolding temperature of BAT5906 is higher than that of Bevacizumab, and thus BAT5906 is more stable than Bevacizumab.

**Table 4. Results of comparison between thermal unfolding temperatures of BAT5906 and control**

| Category | Tm1 (°C) | Tm2 (°C) |
|---|---|---|
| BAT5906 | 76.07 | 83.68 |
| Bevacizumab | 73.11 | 82.12 |

## Example 9: Experiment on Cytobiological Activity Assay

VEGF has the activity of promoting the proliferation of HUVECs, while the monoclonal antibody of the present invention has the function of inhibiting VEGF, so this assay used HUVECs to compare cytobiological activities.

The HUVECs (fifth passage of HUVECs) were first re-suspended with ECM medium containing 2% FBS to reach a concentration of $9 \times 10^4$ cells/mL, then inoculated into a 96-well cell culture plate at 100 $\mu$L/well and incubated for $5\pm1$ h for cell adherence. The monoclonal antibody BAT5906 and Bevacizumab were both pre-diluted to 1000 ng/mL with assay medium and then serially diluted downwards for nine gradients (ten gradients in total) at a ratio of 1:1.5. After the serially diluted samples were each mixed with 80 ng/mL of VEGF-A$_{165}$ diluted with equal volume of assay medium, the mixtures were each incubated at 37 °C for $60\pm10$ min. After the incubation, the antibody-VEGF-A$_{165}$ mixtures were each added at 100 $\mu$L/well into the corresponding wells of the HUVEC-inoculated cell culture plate, and incubation was performed in an incubator under the condition of 5% $CO_2$ for $66\pm3$ h. After the incubation, CCK8 was added at 20 $\mu$L/well, and incubation was continued for $4\pm0.5$ h for chromogenesis. After chromogenesis was completed, the cell culture plate was equilibrated under room temperature for 30 min, and absorbance results were read at 450 nm in a microplate reader. The experiment results are shown in Table 5. The cytobiological activity of BAT5906 was about 3.8 times that of Bevacizumab.

**Table 5. Results of comparison between cytobiological activities of BAT5906 and control**

| Category | Cytobiological activity (EC50) |
|---|---|
| BAT5906 | 0.082 |
| Bevacizumab | 0.313 |

## Example 10: Experiment on Five-week Stability of Diluted Antibody at 37 °C

The changes of stability in eyeballs subsequent to the injection of anti-VEGF monoclonal antibody drugs in human vitreous bodies were simulated by an *in vitro* experiment. The drugs were placed at 37 °C after being diluted according to clinically used concentrations, and their stability was studied. The specific operation was as follows: After being respectively diluted to 0.3125 mg/mL, 0.125 mg/mL, 0.3125 mg/mL and 0.5 mg/mL, the antibody BAT5906 of the present invention, Ranibizumab, Bevacizumab and Aflibercept were placed in a constant-temperature incubator under 37 °C to undergo a stability study for five consecutive weeks. Samples were collected after the first dilution, and then regularly collected every week for later use. On week 5, all the samples at the six time points were detected for cytobiological activities of the monoclonal antibodies and SEC (size exclusion chromatography) main peak percentage changes.

For the method of cytobiological activity experiment, see Example 9. The SEC detection method was as follows: The chromatographic column was TSKgel G3000 SWXL (column specifications: 7.8 × 300 mm, 5 μm), with 100 mM potassium phosphate containing 10% acetonitrile and 125 mM potassium chloride buffer as mobile phase, the column temperature was 30 °C, the flow rate was 0.5 mL/min, and the detection wavelength was 280 nm. Each control was taken and diluted with water into a 2 mg/mL loading solution. 50 μL of the loading solution was injected into a liquid chromatograph, a chromatogram was recorded, and parallel determination was performed three times. The theoretical plate numbers of the main peaks of the three results should not be less than 2000, the tailing factor should not be greater than 2.0, and the resolution between the main peaks and the polymers should not be less than 2.0. The test sample was tested with the same method, calculation was performed according to the area normalization method, and the mean of three detection results was taken as a final detection result. The experiment results are shown as FIG. 8 and FIG. 9.

The experiment results show that the antibody of the present invention, when studied under the conditions of simulating diluted concentration of injection in the human vitreous body and placed at 37 °C, exhibits stable cytobiological activity and small SEC main peak percentage change, indicating that the antibody of the present invention has good stability under the conditions of this experiment, and is an anti-VEGF monoclonal antibody that can exert a stable biological function at the fundus after being injected in the vitreous body.

Although the present invention has been described with reference to the specific embodiments and the examples thereof, it is obvious that many alternative solutions, modifications and variations will be apparent to those skilled in the art. Accordingly, it is intended to cover all such alternative solutions, modifications and variations that fall within the spirit and broad scope of the appended claims.

All publications, patents and patent applications mentioned in this specification are incorporated herein by reference in their entirety as if each publication, patent or patent application was specifically and individually indicated to be incorporated herein by reference.

**Claims**

1.  A full-length humanized anti-VEGF IgG1 antibody, comprising:

    (a) two immunoglobulin light chains; and
    (b) two immunoglobulin heavy chains;

    wherein, the heavy chains comprise an amino acid sequence having at least 80%, at least 85% or at least 90% identity with an amino acid sequence set forth in SEQ ID NO: 1; preferably, the heavy chains comprise an amino acid sequence having at least 91%, at least 92%, at least 93%, at least 94%, at least 95%, at least 96%, at least 97% or at least 98% identity with an amino acid sequence set forth in SEQ ID NO: 1; more preferably, the heavy chains comprise an amino acid sequence having at least 98.1%, at least 98.2%, at least 98.3%, at least 98.4%, at least 98.5%, at least 98.6%, at least 98.7%, at least 98.8% or at least 98.9% identity with an amino acid sequence set forth in SEQ ID NO: 1; further more preferably, the heavy chains comprise an amino acid sequence having at least 99%, at least 99.1%, at least 99.2%, at least 99.3%, at least 99.4%, at least 99.5%, at least 99.6%, at least 99.7%, at least 99.8% or at least 99.9% identity with an amino acid sequence set forth in SEQ ID NO: 1;
    the light chains comprise an amino acid sequence having at least 80%, at least 85% or at least 90% identity with an amino acid sequence set forth in SEQ ID NO: 2; preferably, the light chains comprise an amino acid sequence having at least 91%, at least 92%, at least 93%, at least 94%, at least 95%, at least 96%, at least 97% or at least 98% identity with an amino acid sequence set forth in SEQ ID NO: 2; more preferably, the light chains comprise an amino acid sequence having at least 98.1%, at least 98.2%, at least 98.3%, at least 98.4%, at least 98.5%, at least 98.6%, at least 98.7%, at least 98.8% or at least 98.9% identity with an amino acid sequence set forth in SEQ ID NO: 2; further more preferably, the light chains comprise an amino acid sequence having at least 99%, at least 99.1%, at least 99.2%, at least 99.3%, at least 99.4%, at least 99.5%, at least 99.6%, at least 99.7%, at least 99.8% or at least 99.9% identity with an amino acid sequence set forth in SEQ ID NO: 2;
    preferably, the antibody is human IgG1;
    preferably, the antibody binds to VEGF; more preferably, the antibody binds to VEGF-A; further more preferably, the antibody binds to VEGF-A165;
    preferably, the KD value of the binding between the antibody and human VEGF does not exceed 10 nM; more preferably, the KD value of the binding between the antibody and human VEGF does not exceed 1 nM; and further more preferably, the KD value of the binding between the antibody and human VEGF does not exceed 500 pM.

2.  The antibody of claim 1, wherein the heavy chains comprise an amino acid sequence set forth in SEQ ID NO: 1.

3.  The antibody of claim 1, wherein the light chains comprise an amino acid sequence set forth in SEQ ID NO: 2.

4.  A nucleic acid, encoding the antibody of any one of claims 1-3.

5.  A vector, comprising the nucleic acid of claim 4.

6.  A host cell, comprising the vector of claim 5.

7.  A method for producing the antibody of any one of claims 1-3, comprising culturing the host cell of claim 6 so as to express nucleic acid, and preferably, the method further comprising isolating the antibody from a host or host culture.

8.  An affinity purification reagent, comprising the antibody of any one of claims 1-3.

9.  A use of the antibody of any one of claims 1-3 in the preparation of a reagent for diagnostic analysis of VEGF protein.

10. A kit for diagnosis of VEGF protein, comprising the antibody of any one of claims 1-3.

11. A pharmaceutical preparation, comprising the antibody of any one of claims 1-3, and preferably, further comprising a pharmaceutically acceptable carrier.

12. A use of the antibody of any one of claims 1-3 in the preparation of a medicament for a disease associated with VEGF overexpression in a mammal;
    wherein, preferably, the disease associated with VEGF overexpression is a fundus lesion associated with VEGF overexpression; more preferably, the disease associated with VEGF overexpression is selected from age-related macular degeneration, choroidal neovascularization secondary to pathological myopia, diabetic macular edema, diabetic retinopathy, branch retinal vein occlusion and central retinal vein occlusion;
and preferably, the mammal is human being.

FIG. 1

M: 250 Kda pre-stained protein marker
Lane 1: non-reduced sample of BAT5906

FIG. 2

M: 250 Kda pre-stained protein marker
Lane 1: reduced sample of BAT5906

FIG. 3

FIG. 4

FIG. 5

FIG. 6

| Animal No./eye | 2M001/right eye | |
|---|---|---|
| Time | Early stage | Late stage |
| Before administration | | |
| On day 14 of experiment | | |

FIG. 7

FIG. 8

FIG. 9

<div align="center">**INTERNATIONAL SEARCH REPORT**</div>

| | |
|---|---|
| International application No. |
| **PCT/CN2019/070479** |

**A.   CLASSIFICATION OF SUBJECT MATTER**

C07K 16/22(2006.01)i;   C07K 16/24(2006.01)i;   A61K 39/395(2006.01)i;   A61P 27/02(2006.01)n

According to International Patent Classification (IPC) or to both national classification and IPC

**B.   FIELDS SEARCHED**

Minimum documentation searched (classification system followed by classification symbols)

C07K,A61K,A61P

Documentation searched other than minimum documentation to the extent that such documents are included in the fields searched

Electronic data base consulted during the international search (name of data base and, where practicable, search terms used)

CNABS, CNTXT, TWTXT: 吴晓云, 百奥泰生物科技, VEGF-A, 抗体, 单克隆抗体, 人源化, 眼, 黄斑变性, 视网膜: VEN, WOTXT, EPTXT, USTXT: VEGF-A, ANTIBODY, MAB, HUMANIZE?, EYE?, MACULAR DEGENERATION, RETINA GENBANK DATABASE, 中国专利生物序列检索系统: SEQ ID NO: 1, 2, China Patents Sequence Search System: SEQ ID NOs: 1, 2

**C.   DOCUMENTS CONSIDERED TO BE RELEVANT**

| Category* | Citation of document, with indication, where appropriate, of the relevant passages | Relevant to claim No. |
|---|---|---|
| X | CN 107428824 A (KODIAK SCIENCES INC.) 01 December 2017 (2017-12-01) description, paragraphs 8, 9, 36, 37, 208, 225, 235-238, 353, 369-377, and figures 5, 8A, and 8B | 1-12 |
| X | CN 104428315 A (ROCHE GLYCART AG) 18 March 2015 (2015-03-18) description, paragraphs 4-66 and 226-430 | 1-12 |
| X | CN 105079804 A (BIO-THERA SOLUTIONS, LTD.) 25 November 2015 (2015-11-25) description, paragraphs 3-102 | 1-12 |
| X | CN 100480269 C (GENENTECH INC.) 22 April 2009 (2009-04-22) description, p. 4, paragraph 1 to p. 35, paragraph 6, and figures 1A, 1B, 10A, and 10B | 1-12 |

☐ Further documents are listed in the continuation of Box C.       ☑ See patent family annex.

| | |
|---|---|
| \*   Special categories of cited documents:<br>"A"   document defining the general state of the art which is not considered to be of particular relevance<br>"E"   earlier application or patent but published on or after the international filing date<br>"L"   document which may throw doubts on priority claim(s) or which is cited to establish the publication date of another citation or other special reason (as specified)<br>"O"   document referring to an oral disclosure, use, exhibition or other means<br>"P"   document published prior to the international filing date but later than the priority date claimed | "T"   later document published after the international filing date or priority date and not in conflict with the application but cited to understand the principle or theory underlying the invention<br>"X"   document of particular relevance; the claimed invention cannot be considered novel or cannot be considered to involve an inventive step when the document is taken alone<br>"Y"   document of particular relevance; the claimed invention cannot be considered to involve an inventive step when the document is combined with one or more other such documents, such combination being obvious to a person skilled in the art<br>"&"   document member of the same patent family |

| Date of the actual completion of the international search | Date of mailing of the international search report |
|---|---|
| | **19 March 2019** |

| Name and mailing address of the ISA/CN | Authorized officer |
|---|---|
| **State Intellectual Property Office of the P. R. China (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.

**PCT/CN2019/070479**

| Patent document cited in search report | | | Publication date (day/month/year) | Patent family member(s) | | | Publication date (day/month/year) |
|---|---|---|---|---|---|---|---|
| CN | 107428824 | A | 01 December 2017 | JP | 2017526635 | A | 14 September 2017 |
| | | | | EP | 3161000 | A2 | 03 May 2017 |
| | | | | CA | 2953698 | A1 | 30 December 2015 |
| | | | | EP | 3161000 | A4 | 02 May 2018 |
| | | | | AU | 2015279560 | A1 | 19 January 2017 |
| | | | | MX | 2016017290 | A | 24 August 2017 |
| | | | | WO | 2015200905 | A3 | 18 February 2016 |
| | | | | WO | 2015200905 | A2 | 30 December 2015 |
| | | | | HK | 1237353 | A0 | 13 April 2018 |
| | | | | US | 2015376271 | A1 | 31 December 2015 |
| | | | | US | 2018244762 | A1 | 30 August 2018 |
| | | | | US | 9840553 | B2 | 12 December 2017 |
| CN | 104428315 | A | 18 March 2015 | HR | P20181595 | T1 | 14 December 2018 |
| | | | | SI | 2872534 | T1 | 30 November 2018 |
| | | | | TW | I506036 | B | 01 November 2015 |
| | | | | AR | 092027 | A1 | 18 March 2015 |
| | | | | MX | 2014015518 | A | 23 June 2015 |
| | | | | CR | 20140542 | A | 12 February 2015 |
| | | | | CL | 2015000061 | A1 | 08 May 2015 |
| | | | | SG | 11201408538P | A | 27 February 2015 |
| | | | | EP | 2872534 | B1 | 08 August 2018 |
| | | | | LT | 2872534 | T | 25 October 2018 |
| | | | | DK | 2872534 | T3 | 22 October 2018 |
| | | | | AU | 2013288641 | A1 | 11 December 2014 |
| | | | | PL | 2872534 | T3 | 31 December 2018 |
| | | | | HK | 1206365 | A1 | 08 January 2016 |
| | | | | JP | 2015527064 | A | 17 September 2015 |
| | | | | US | 2014017244 | A1 | 16 January 2014 |
| | | | | EA | 201500132 | A1 | 31 August 2015 |
| | | | | TW | 201406782 | A | 16 February 2014 |
| | | | | MA | 37794 | A1 | 31 March 2016 |
| | | | | US | 2017260265 | A1 | 14 September 2017 |
| | | | | AU | 2013288641 | B2 | 06 July 2017 |
| | | | | CN | 104428315 | B | 29 September 2017 |
| | | | | JP | 6154900 | B2 | 28 June 2017 |
| | | | | IN | 11157DEN2014 | A | 02 October 2015 |
| | | | | CA | 2874554 | A1 | 16 January 2014 |
| | | | | PH | 12014502713 | A1 | 02 February 2015 |
| | | | | IL | 236292 | A | 26 February 2015 |
| | | | | RS | 57704 | B1 | 31 December 2018 |
| | | | | KR | 20150023688 | A | 05 March 2015 |
| | | | | US | 9695233 | B2 | 04 July 2017 |
| | | | | PE | 03612015 | A1 | 14 March 2015 |
| | | | | ES | 2690312 | T3 | 20 November 2018 |
| | | | | CL | 2017002146 | A1 | 13 April 2018 |
| | | | | EP | 2872534 | A1 | 20 May 2015 |
| | | | | KR | 101774121 | B1 | 01 September 2017 |
| | | | | CO | 7151542 | A2 | 29 December 2014 |
| | | | | JP | 2017140038 | A | 17 August 2017 |
| | | | | MA | 37794 | B1 | 31 July 2017 |

Form PCT/ISA/210 (patent family annex) (January 2015)

<div align="center">

**INTERNATIONAL SEARCH REPORT**
**Information on patent family members**

</div>

| International application No. |
| --- |
| **PCT/CN2019/070479** |

| Patent document cited in search report | | | Publication date (day/month/year) | Patent family member(s) | | | Publication date (day/month/year) |
| --- | --- | --- | --- | --- | --- | --- | --- |
| | | | | NZ | 702201 | A | 26 January 2018 |
| | | | | WO | 2014009465 | A1 | 16 January 2014 |
| | | | | IL | 236292 | D0 | 26 February 2015 |
| | | | | BR | 112015000800 | A2 | 15 August 2017 |
| | | | | GC | 8199 | A | 31 July 2018 |
| | | | | ID | 201700759 | A | 10 February 2017 |
| | | | | IL | 236292 | A1 | 29 January 2015 |
| | | | | IN | 2014DELNP11157 | | 29 December 2014 |
| | | | | SG | 2014011008538 | | 11 July 2013 |
| | | | | SG | 11201408538 | B | 27 February 2015 |
| | | | | VN | 42828 | A | 27 July 2015 |
| CN | 105079804 | A | 25 November 2015 | None | | | |
| CN | 100480269 | C | 22 April 2009 | JP | 3957765 | B2 | 15 August 2007 |
| | | | | AT | 349470 | T | 15 January 2007 |
| | | | | CN | 1259962 | A | 12 July 2000 |
| | | | | DE | 122005000050 | I1 | 29 December 2005 |
| | | | | ES | 2236634 | T3 | 16 July 2005 |
| | | | | NO | 2007014 | I1 | 14 January 2008 |
| | | | | EP | 1325932 | A2 | 09 July 2003 |
| | | | | ES | 2273415 | T3 | 01 May 2007 |
| | | | | NO | 994869 | A | 06 December 1999 |
| | | | | EP | 1787999 | A1 | 23 May 2007 |
| | | | | EP | 0973804 | A2 | 26 January 2000 |
| | | | | TR | 9903123 | T2 | 22 May 2000 |
| | | | | SI | EP1325932 | T1 | 31 August 2005 |
| | | | | NO | 2007014 | I2 | 21 February 2011 |
| | | | | EP | 1650220 | A2 | 26 April 2006 |
| | | | | EP | 1325932 | A3 | 29 October 2003 |
| | | | | NL | 300193 | I1 | 01 July 2005 |
| | | | | NO | 2007015 | I1 | 14 January 2008 |
| | | | | LU | 91167 | I2 | 20 June 2005 |
| | | | | KR | 100816621 | B1 | 24 March 2008 |
| | | | | TR | 199903123 | T2 | 22 May 2000 |
| | | | | EP | 1325932 | B9 | 19 July 2006 |
| | | | | EP | 1650220 | A3 | 03 May 2006 |
| | | | | NL | 300193 | I2 | 03 October 2005 |
| | | | | DE | 122005000026 | I1 | 04 August 2005 |
| | | | | US | 7060269 | B1 | 13 June 2006 |
| | | | | NO | 2007015 | I2 | 28 November 2011 |
| | | | | DK | 1325932 | T5 | 03 October 2005 |
| | | | | LU | 91320 | I9 | 31 December 2018 |
| | | | | DE | 69836729 | D1 | 08 February 2007 |
| | | | | KR | 20080003452 | A | 07 January 2008 |
| | | | | NO | 994869 | D0 | 06 October 1999 |
| | | | | CA | 2286330 | A1 | 15 October 1998 |
| | | | | IL | 132240 | D0 | 19 March 2001 |
| | | | | CA | 2286330 | C | 10 June 2008 |
| | | | | DK | 0973804 | T3 | 07 May 2007 |
| | | | | DK | 1325932 | T3 | 30 May 2005 |
| | | | | DE | 69829891 | D1 | 25 May 2005 |

Form PCT/ISA/210 (patent family annex) (January 2015)

**INTERNATIONAL SEARCH REPORT**
Information on patent family members

International application No.

**PCT/CN2019/070479**

| Patent document cited in search report | Publication date (day/month/year) | Patent family member(s) | | Publication date (day/month/year) |
|---|---|---|---|---|
| | | NL | 300278 I1 | 01 June 2007 |
| | | SI | 1325932 T1 | 31 August 2005 |
| | | HK | 1023577 A1 | 30 March 2007 |
| | | LT | PA2005005 I1 | 27 November 2017 |
| | | EP | 0973804 B1 | 27 December 2006 |
| | | NL | 300278 I2 | 01 October 2007 |
| | | EP | 1325932 B1 | 20 April 2005 |
| | | AT | 293640 T | 15 May 2005 |
| | | PT | 1325932 T | 30 June 2005 |
| | | BR | PI9809387 B1 | 22 November 2016 |
| | | JP | 2001509817 A | 24 July 2001 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KABAT et al.** Sequences of Proteins of Immunological Interest. National Institutes of Health, 1991, vol. 15
- **GRAHAM, F.L. ; VAN DER EB, A.J.** *Virology,* 1973, vol. 52, 546-467
- **COHEN, S.N. et al.** *PNAS (Proceedings of the National Academy of Sciences),* 1972, vol. 69, 2110-2114
- **GAUDREAULT J ; FEI D ; RUSIT J et al.** Preclinical pharmacokinetics of Ranibizumab (rhuFabV2) after a single intravitreal administration[J. *Investigative ophthalmology & visual science,* 2005, vol. 46 (2), 726-733
- Biologic License Application (BLA):125387 Orig1s000. *PHARMACOLOGY REVIEW(S), Company: REGENERON PHARMACEUTICALS,* 18 November 2011